# EUROPEAN PATENT SPECIFICATION

(11) **EP 0 365 858 B1**
(45) Date of publication and mention of the grant of the patent: **30.11.1994**
(21) Application number: 89117873.3
(22) Date of filing: 27.09.1989
(51) Int. Cl.: C07K 3/20, C07K 3/22, C07K 13/00

(54) **Process for reducing the aggregate content of growth hormone-like materials**
Verfahren zur Verringerung des Aggregatinhaltes in Wachstumshormonen gleichenden Stoffen
Procédé pour réduire le contenu agrégat de matériaux semblables à l'hormone de croissance

(30) Priority: 26.10.1988 US 262855
(43) Date of publication of application: 02.05.1990
(73) Proprietor: AMERICAN CYANAMID COMPANY, Wayne, NJ 07470-8426 (US)
(72) Inventor: Simpson, John McLean, Upper Nyack New York 10960 (US); McCoy, Kevin Michael, Hoboken New Jersey 07030 (US)
(74) Representative: Wächtershäuser, Günter, Prof. Dr.

(56) References cited:
- EP-A- 0 131 843
- EP-A- 0 218 374

## Description

### Summary of the Invention

The invention is an improvement in a flow-through chromatographic method employing a strong anion exchanger-type media for the separation of covalent and non-covalent aggregates from monomeric growth hormone-like material which comprises: equilibrating the media in a starting buffer species with a pKa near 11.5 and a sufficient concentration of a simple salt to cause the monomeric growth hormone-like material not to be adsorbed to the media; adding the proper amount of the buffer species and simple salt to the growth hormone-like material solution to be purified such that the concentration of each is the same as the starting buffer; loading the growth hormone-like material solution onto a column packed with media and collecting the flow-through effluent from the feed solution and from a displacement wash using the starting buffer (the effluent contains the monomeric growth hormone-like material); and washing the column media with high salt and/or caustic solutions to strip the media of strongly adsorbed species and then regenerating the media for the next cycle; the improvement which comprises carrying out the method wherein the pH of the solution is above 10.5. The invention includes an adsorption/desorption elution chromatographic method employing a strong anion exchanger-type media for the separation of covalent and non-covalent aggregates from adsorption/desorption elution chromatographic method employing a strong anion exchanger-type media for the separation of covalent and non-covalent aggregates from monomeric growth hormone-like material which comprises; equilibrating the media in a starting buffer with a pKa near 11.5 and a suitable concentration of a simple salt; adding the proper amount of the buffer species and simple salt to the growth hormone-like material solution to be purified such that the concentration of each is the same as the starting buffer; loading the growth hormone-like material solution onto a column packed with media followed by a displacement wash with the starting buffer; eluting the monomeric growth hormone-like material using an isocratic or gradient methodology; and washing and regenerating the media with suitable buffers to remove strongly adsorbed species and prepare the media for the next cycle.

### The Drawings

Figure 1 is the SDS-PAGE of starting material (lane 1) and chromatographed material (lane 2) using the methodology of Example 1.

Figure 2 is the gel permeation chromatographs using a Superose 6 (Pharmacia) column. The top chromatogram is the feed solution while the bottom is the lyophilized effluent from the anion exchanger as described in Example 2.

Figure 3 is the chromatogram obtained using the elution method described in Example 3. The column used was 1-cc bed volume of Mono-Q anion-exchanger (Pharmacia).

### Detailed Description of the Invention

Recombinant DNA technology has made it possible to produce large quantities of proteins for potential commercial uses which were previously implausible because of the limited availability of such proteins from natural sources. However, the proteins produced by microorganisms which have been genetically manipulated are often not correctly folded, and thus are biologically inactive.

Recently, there have been several disclosures regarding the recovery, isolation, and solubilization of inclusion-body proteins from bacteria. The objective of these disclosures has been to describe processes which maximize the conversion of denatured insoluble protein to the properly folded, monomeric, biologically active form. Inevitably, improperly folded non-covalent aggregates or covalent oligomers are produced during the course of solubilization and renaturation. These products are not biologically active and must be separated from the desired bioactive monomeric protein.

Accordingly, it is the object of this invention to provide an efficient method(s) for the separation of these biologically inactive aggregate and/or oligomer impurities from the monomeric bioactive protein.

This invention discloses an efficient and economical method(s) for separating high molecular weight impurities from monomeric growth hormone-like materials. The high molecular weight impurities may be the result of intermolecular covalent bonding or non-covalent association. The chromatographic method(s) described efficiently separate such covalent or non-covalent aggregates which are biologically inactive from the monomeric bioactive protein.

The method of invention employs a chromatographic procedure(s) based on separation by ionic mechanisms, i.e., ion-exchange chromatography, which is far more efficient and economical than conventional means of separating aggregates from monomer such as gel permeation chromatography. The advantages of ion-exchange chromatography include higher protein loading per unit volume of media, higher linear velocity through the media without loss of resolution or excessive back pressure, and the ability to remove bacterial host impurities such as endotoxins. The first three aforementioned advantages result in capital and operating cost savings for a commercial-scale production facility by reducing the size of equipment and amount of chromatographic media needed for a fixed production rate. The last stated advantage allows the elimination of a separate unit operation by combining the functions of two operations into one.

The material that is produced by the disclosed chromatographic method(s) contains very low amounts of aggregate impurities. Similar purities may be obtained through extensive processing by known techniques. It is the object of this invention to provide a much more efficient and inexpensive alternative to producing materials which are primarily composed of bioactive monomeric protein. The advantages of the invention for a commercial-scale production facility will be readily apparent to those skilled in the art from the following description and examples.

### Description

For purposes of this specification the term "growth hormone-like material" as used herein denotes (1) animal growth hormone itself, derivatives, analogs and fragments thereof of whatever species, for example, human, bovine, or porcine; (2) precursors to growth hormone, as reduced (-SH) growth hormone and S-protected growth hormone, for example, growth hormone S-sulfonate; (3) variants of growth hormone or its precursors, for exmaple, structures which have been modified to lengthen and/or shorten the growth hormone amino acid sequence, for example the 20K variant of human growth hormone, methionyl human growth hormone, and the like; and (4) analogs of growth hormone or its precursors, for example structures in which the growth hormone amino acid sequence has been modified by replacement of one or more amino acid residues.

This invention is concerned with the description of chromatographic method(s) to be used for the separation of aggregates of growth hormone-like materials from the desired bioactive monomeric material. Prior disclosures have adequately described the recovery, isolation, and solubilization of inclusion bodies from the host bacterium. Due to the inevitable inefficiencies of the solubilization method some improperly folded non-covalent aggregates or covalent oligomers are produced with the desired monomer. Ultrafiltration may be employed to remove colloids and very high molecular weight impurities, however a substantial quantity of aggregate usually passes through the ultrafilter with the monomer. This ultrafiltrate may be advantageously purified by the following chromatographic method.

Those skilled in the art will recognize that the term "strong anion-exchanger" refers to resins having cationic groups which maintain their positive charge at relatively high pH, e.g. above pH 9.0. The cationic groups attached to the support may be selected from quaternary ammonium groups, such as --CH₂CH₂N+(CH₂CH₃)₂CH₂CH(OH)CH₃. One can mention as examples of suitable commercially available anion-exchange resins for use in the practice of the invention QAE-Sephadex A-25, QE-52 cellulose, Cellex QAE, Q-Sepharose, and Mono-Q.

The ion-exchange media employed is a strong anion exchanger type such as, but not limited to, Q-Sepharose (Pharmacia). The media is packed into a conventional chromatography column that is part of a typical chromatographic system package consisting pumps, buffer reservoirs, uv detector, and fraction collector. The media is equilibrated in a starting aqueous buffer consisting of piperidine or another suitable buffering agent with a similar pKa, at a concentration of 20 mM and sodium chloride, or a similar simple salt, at a concentration of 50-250 mM. The preferred pH of the solution is 11.3-11.7 and more or less piperidine may be used to achieve the desired pH of the starting buffer. Three bed volumes of buffer are usually adequate to ensure equilibration.

The solution containing the soluble monomeric growth hormone-like material and impurities, herein referred to as the feed solution, is brought to the same piperidine and sodium chloride concentration as the starting buffer. The pH is adjusted to achieve the desired result by adding more piperidine if necessary. The feed solution is then loaded onto the column at a superficial velocity of up to 300 cm-hr⁻¹ until 50-150 grams of protein have been loaded per liter of media. The effluent is monitored by absorbance at 280 nm and fractions are collected once a rise in the baseline occurs. The feed solution is followed by 3-6 bed volumes of the starting buffer until baseline is reached once again. All fractions collected from the initial breakthrough until baseline is reached are pooled together. The column may be regenerated for further use by washing it with suitable buffers to remove strongly bound impurities and re-equilibrating in the starting buffer.

It has been demonstrated that the monomeric form of the growth hormone-like material does not bind to the active ion-exchange sites on the media, but instead flows through the column and is recovered in the effluent. However, the aggregate impurities do bind to the column and are retained until a suitable higher strength displacer is used to elute them.

In addition to the flow-through method described above it has been demonstrated that an adsorption/desorption elution chromatographic method is also effective for the separation of aggregate impurities from monomeric growth hormone-like material. The elution chromatographic method is however, less efficient at similar protein loadings per unit volume of media. Nevertheless, at lower protein loadings it still is a viable method for separating monomer from aggregates.

The ion-exchange media and associated equipment used for the elution chromatographic method is the same as for the flow-through method. The buffers used are aqueous buffer (A) composed of 20 mM piperidine, or another suitable buffering agent with a similar pKa, to give a pH of 11.3-11.7 and aqueous buffer (B) composed of 20 mM piperidine and 1000 mM sodium chloride, or a similar simple salt. The column is first equilibrated using 17% v/v of buffer (B) in buffer (A). Three bed volumes are usually sufficient to ensure equilibration. Secondly, the feed solution consisting of soluble monomeric growth hormone-like material and impurities is brought to the same composition as buffer (A) and then fed to the column until 5-15 grams of protein has been loaded per liter of media. This is followed by a displacement wash consisting of five bed volumes of 17% v/v of buffer (B) in buffer (A). The elution is accomplished using 20 bed volumes of a linear gradient beginning with 17% v/v of buffer (B) in buffer (A) to 47% v/v of buffer (B) in buffer (A). The eluate is monitored by absorbance at 280 nm and fractions are collected. Lastly, ten bed volumes of buffer (B) is used to strip the column of any strongly adsorbed species. The column may then be regenerated for further use by additional washings with suitable buffers to remove other strongly bound impurities and re-equilibrating with the starting buffer.

It has been demonstrated that the monomeric form of the growth hormone-like material is eluted early on in the course of the elution salt gradient and that the aggregate impurities are eluted later in the gradient with baseline resolution between the two-peaks.

The above-described chromatographic methods have been shown to be effective for the separation of monomer from aggregate impurities using relatively crude protein solutions, i.e., high levels of impurities relative to the desired monomer. The methods have also, as might be imagined, been successfully used to further purify or rework relatively clean protein solutions which have already been through several purification steps. The flow-through method is especially suited for economical reworking of already purified protein which might contain aggregate levels only slightly higher than specification.

The following examples are intended to further illustrate the practice of the invention but not to limit its scope.

### Example 1

This example describes the flow-through methodology used to further purify of rework bovine somatotropin derived from recombinant bacteria that has already been partially purified, but contains a high amount of aggregate.
a column containing 200 cc of Q-Sepharose (Pharmacia) anion-exchanger gel was equilibrated using three bed volumes of starting aqueous buffer consisting of 20 mM piperidine and 150 mM sodium chloride with a pH of 11.5. Twenty grams of lyophilized recombinant bovine somatotropin from the American Cyanamid Company was reconstituted in 2000 cc of starting buffer to give a concentration of 10 grams per liter and loaded onto the column at a superficial linear velocity of 95 cm-hr⁻¹. Soon after loading was begun breakthrough was observed by monitoring the absorbance of the effluent at 280 nm. The effluent was collected once breakthrough occurred. The feed solution was followed by a displacement wash of the same composition as the starting buffer until the absorbance had returned to baseline. the effluent from the loading phase after breakthrough was combined with the effluent from the displacement wash to give a total volume of 2840 cc. The combined effluent was desalted and concentrated using a 10K Dalton cutoff hollow-fiber cartridge and then lyophilized.

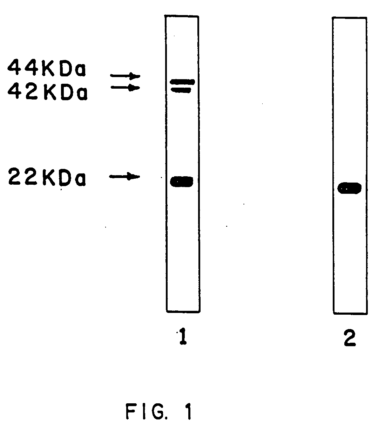

The starting material in this example contained 11% aggregate by non-reducing SDS-PAGE all of which had an apparent molecular weight of 44K or 42K Daltons. This material is believed to be covalent dimer formed by intermolecular disulfide bridging between bovine somatotropin monomers. It has also been shown that the covalent dimer is not biologically active. The material obtained after chromatography had 0.2% dimer by SDS-PAGE. The aggregate level, as measured by size-exclusion chromatography, also showed a decrease from over 10% aggregate to less than 1% between starting and chromatographed material. On a monomer-only basis the yield across the chromatography step was 84%, i.e., 84% of the monomer in the feed was recovered in the combined effluent. In addition the bioactivity of the material was increased from 92 to 101% of standard as measured by a radio-receptor assay. The results of an analytical comparison between the starting material and the chromatographed material was summarized in Table I. Figure 1 also shows the SDS-PAGE gel lanes for the starting material and the chromatographed material for comparison. Isoelectric focusing was performed on both the starting material and the chromatographed material. The results (not shown) indicated that no deamidation had occurred due to the chromatography step.

**Table 1**

| | Starting Material | Chromatographed Material |
|---|---|---|
| Protein, mg/g | 1011 | 1008 |
| SDS-PAGE, wt. %* | 95 | 104 |
| SDS-PAGE, % dimer* | 11.0 | 0.2 |
| Superose 12® , wt. %** | 99 | 98 |
| Superose 12® , % dimer** | 10.4 | <1.0 |
| Radio-Receptor assay, wt. % | 92 | 101 |

| | | |
|---|---|---|
| * SDS-PAGE = sodium dodecylsulfonate polyacrylamide gel electrophoresis assay. | | |
| **Superose 12 (Pharmacia) = gel permeation chromatography assay. | | |
| NOTE: All assays are calculated on a moisture-free basis. | | |

### Example 2

This example describes the flow-through methodology used to chromatographically purify a relatively crude protein solution which has a substantial quantity of aggregate ranging in size from approximately 40K to over 100K Daltons.
the material to be purified was a crude protein solution of recombinant bovine somatotropin that had been derived from inclusion bodies and subjected to ultrafiltration to remove colloids and very high molecular weight impurities. the solution was obtained from the American Cyanamid Company pilot plant facility.

A column containing 135 cc of Q-Sepharose (Pharmacia) anion-exchanger was equilibrated using the same buffer as described in Example 1. the crude protein solution which had been previously ultrafiltered using a 100K Dalton cut-off hollow-fiber ultrafilter (Amicon) was brought to the same buffer salt composition as the starting equilibration buffer by adding piperidine and sodium chloride. The protein concentration of the solution was 4.71 g/L. The column was loaded with 3180 cc of solution and the effluent was collected as described in Example 1. The feed solution was followed by a displacement wash as described in Example 1. The total effluent volume collected was 3489 cc. The effluent was desalted, concentrated, and lyophilized to yield a dry weight of 11.4 grams.

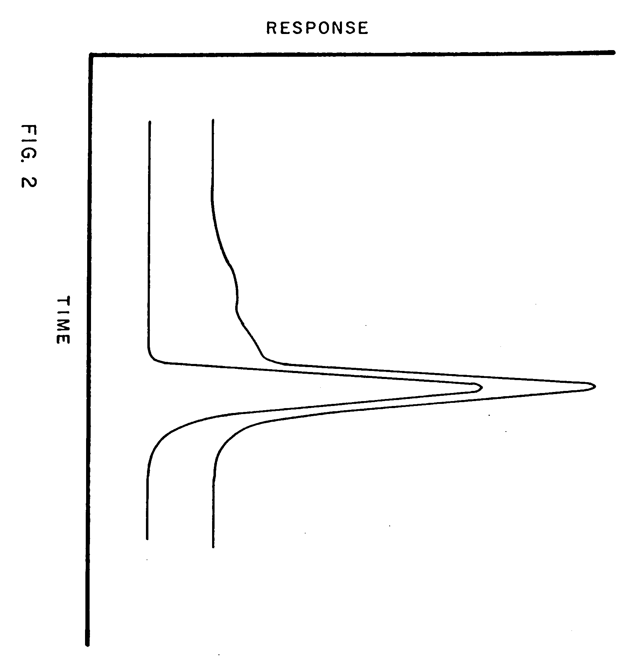

The crude protein solution contained 19.2% aggregate as measured by gel permeation chromatography (gpc). The lyophilized chromatographed material contained less than 1% aggregate by gpc and 0.3% oligomer by SDS-PAGE. Figure 2 shows the gpc chromatograms for the feed solution and the lyophilized effluent from the column.

### Example 3

The present example illustrates the procedures used to chromatographically purify a sample of recombinant bovine somatotropin using the adsorption/desorption elution method. The material was obtained from the American Cyanamid Company and contained greater than 10% aggregate as measured by SDS-PAGE.

A 1-cc Mono-Q (Pharmacia) anion-exchanger column was used for the present example. The buffers were aqueous buffer (A) composed of 20 mM piperidine, pH 11.5, and aqueous buffer (B) composed of 20 mM piperidine, 1000 mM sodium chloride, pH 11.5. The column was equilibrated using 17% v/v of buffer (B) in buffer (A) and then 200 microliters of a 20 mg/mL solution of bovine somatotropin in buffer (A) was injected. The injection was followed by five bed volumes of 17% v/v of buffer (B) in buffer (A) and then by 20 bed volumes of a linear gradient from 17% to 47% v/v of buffer (B) in buffer (A). Finally, ten bed volumes of 100% buffer (B) was used to elute any strongly adsorbed impurities.

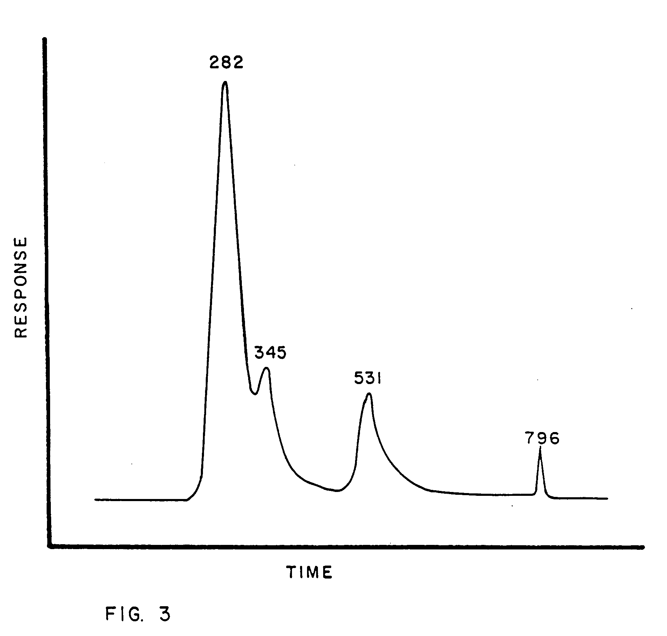

Three well-defined peaks were obtained during the salt gradient (see Figure 3). The first peak (282 seconds), which appeared shortly after the start of the gradient, was the largest. The second appeared as a fused peak (345 seconds) on the trailing edge of the first. The third peak (531 seconds) was well separated from the first two. In addition, a small peak (796 seconds) was eluted during the isocratic 100% buffer (B) wash.

The individual peaks were isolated and examined by SDS-PAGE. The first and second peaks were found to be pure bovine somatotropin monomer. The third peak however, was composed of bovine somatotropin dimer and higher molecular weight oligomers.

### Example 4

This example details the procedures used to further purify porcine somatotropin derived from recombinant bacterial that has already been partially purified, but contained 22.4% aggregate as determined by gel permeation chromatography.

A column containing 4.5 L of Q-Sepharose (Pharmacia) anion-exchanger gel was used in the flow-through mode according to the same procedures described in Example 1. Buffer volumes were adjusted to accommodate the difference in bed volumes between the two examples. A total of 450 grams of lyophilized recombinant porcine somatotropin obtained from the American Cyanamid Company was reconstituted in 45 L of the starting buffer and then fed to the column. The effluent was monitored and collected as described in Example 1. The displacement wash effluent was combined with the feed period effluent as previously detailed in Example 1.

The bulk combined effluent was shown to contain a non-detectable amount of aggregate as determined by gpc.

## Claims

1. In a flow-through chromatographic method employing a strong anion exchanger-type media for the separation of covalent and non-covalent aggregates from monomeric growth hormone-like material which comprises: equilibrating the media in a starting buffer species with a pKa near 11.5 and a sufficient concentration of a simple salt to cause the monomeric growth hormone-like material not to be absorbed to the media; adding the proper amount of the buffer species and simple salt to the growth hormone-like material solution to be purified such that the concentration of each is the same as the starting buffer; loading the growth hormone-like material solution onto a column packed with media and collecting the flow-through effluent from the feed solution and from a displacement wash using the starting buffer; and washing the column media with high salt and/or caustic solutions to strip the media of strongly absorbed species and then regenerating the media for the next cycle;
the improvement which comprises carrying out the method wherein the pH of the solution is above 10.5.

2. The method of claim 1 wherein the pH of the solution is 11.3 - 11.7 and the simple salt is sodium chloride at a concentration of 50-250 mM in the starting buffer.

3. The method of claim 2 wherein the buffer species is piperidine or a phosphate salt.

4. The method of claim 3 wherein the growth hormone-like material is recombinant or pituitary bovine or porcine somatotropin.

5. The method of claim 4 wherein the solution of growth hormone-like material to be purified is crude (high levels of impurities) or is relatively pure (low impurity levels).

6. In an absorption/desorption elution chromatographic method employing a strong anion exchanger-type media for the separation of covalent and non-covalent aggregates from monomeric growth hormone-like material which comprises: equilibrating the media in a starting buffer species with a pKa near 11.5 and a suitable concentration of a simple salt; adding the proper amount of the buffer species and simple salt to the growth hormone-like material solution to be purified such that the concentration of each is the same as the starting buffer; loading the growth hormone-like material solution onto a column packed with media followed by a displacement wash with the starting buffer; eluting the monomeric growth hormone-like material using an isocratic or gradient methodology; and washing and regenerating the media with suitable buffers to remove strongly absorbed species and prepare the media for the next cycle;
the improvement which comprises carrying out the method wherein the pH of the solution is above 10.5.

7. The method of claim 6 wherein the pH of the solution is 11.3 - 11.7 and the simple salt is sodium chloride at a concentration of 50-1000 mM.

8. The method of claim 7 wherein the buffer species is piperidine or a phosphate salt.

9. The method of claim 8 wherein the growth hormone-like material is recombinant or pituitary bovine or porcine somatotropin.

10. The method of claim 9 wherein the solution of growth hormone-like material to be purified is crude (high levels of impurities) or is relatively pure (low impurity levels).

## Patentansprüche

1. Durchflußchromatographisches Verfahren unter Anwendung eines Mediums vom starken Anionenaustauschertyp zur Trennung von kovalenten und nichtkovalenten Aggregationen aus monomerem wachstumshormonähnlichem Material, umfassend: Insgleichgewichtbringen des Mediums in einer Startpufferspezies mit einem pKa-Wert nahe 11,5 und einer ausreichenden Konzentration eines Einfachsalzes, um das monomere wachstumshormonähnliche Material nicht an dem Medium absorbieren zu lassen; Zugabe der geeigneten Menge an Pufferspezies und Einfachsalz zu der wachstumshormonähnliches Material enthaltenden Lösung, die zu reinigen ist, derart, daß die Konzentration von jedem die gleiche ist wie die des Startpuffers; Aufgeben der wachstumshormonähnliches Material enthaltenden Lösung auf eine mit dem Medium gefüllten Säule und Sammeln von Durchfluß herrührenden Ausflusses aus der Zuführungslösung und aus einer Verdrängungswaschlösung unter Verwendung des Startpuffers und Waschen des Säulenmediums mit stark salzhaltigen und/oder alkalischen Lösungen zum Abstreifen von stark absorbierenden Spezies von dem Medium und anschließend Regenerieren des Mediums für den nächsten Zyklus; dadurch gekennzeichnet, daß das Verfahren die Ausführung bei einem pH-Wert der Lösung oberhalb 10,5 umfaßt.

2. Verfahren nach Anspruch 1, wobei der pH-Wert der Lösung 11,3 - 11,7 beträgt und das Einfachsalz Natriumchlorid ist bei einer Konzentration von 50 - 250 mM in dem Startpuffer.

3. Verfahren nach Anspruch 2, wobei die Pufferspezies Piperidin oder ein Phosphatsalz ist.

4. Verfahren nach Anspruch 3, wobei das wachstumshormonähnliche Material rekombinantes oder Hypophysen-Rinder- oder Schweinesomatotropin ist.

5. Verfahren nach Anspruch 4, wobei die Lösung des zu reinigenden wachstumshormonähnlichen Materials roh ist, (starke Anteile Verunreinigungen) oder relativ rein ist (geringe Verunreinigungsanteile).

6. Absorptions/Desorptions-elutionschromatographisches Verfahren unter Anwendung eines Mediums vom starken Anionenaustauschertyp zur Trennung von kovalenten und nichtkovalenten Aggregationen aus monomerem wachstumshormonähnlichem Material, umfassend Insgleichgewichtbringen des Mediums in einer Startpufferspezies mit einem pKa nahe 11,5 und einer geeigneten Konzentration eines Einfachsalzes; Zugabe der geeigneten Menge von der Pufferspezies und dem Einfachsalz zu der zu reinigenden wachstumshormonähnliches Material enthaltenden Lösung, derart, daß die Konzentration von jedem dieselbe ist wie die des Startpuffers; Auftragen der wachstumshormonähnliches Material enthaltenden Lösung auf eine mit Medium gefüllte Säule, gefolgt von Verdrängungswaschen mit dem Startpuffer; Eluieren des monomeren wachstumshormonähnlichen Materials unter Verwendung einer isokratischen oder Gradientenmethode und Waschen und Regenerieren des Mediums mit geeigneten Puffern zur Entfernung stark absorbierter Spezies und Zubereiten des Mediums für den nächsten Zyklus;
dadurch gekennzeichnet, daß das Verfahren die Ausführung bei einem pH-Wert der Lösung oberhalb 10,5.

7. Verfahren nach Anspruch 6, wobei der pH-Wert der Lösung 11,3 - 11,7 beträgt und das Einfachsalz Natriumchlorid bei einer Konzentration von 50 - 1000 mM ist.

8. Verfahren nach Anspruch 7, wobei die Pufferspezies Piperidin oder ein Phosphatsalz ist.

9. Verfahren nach Anspruch 8, wobei das wachstumshormonähnliche Material rekombinantes oder Hypophysen-Rinder- oder Schweinesomatotropin ist.

10. Verfahren nach Anspruch 9, wobei die Lösung des zu reinigenden wachstumshormonähnlichen Materials roh ist (hohe Anteile an Verunreinigungen) oder relativ rein ist (geringe Anteile an Verunreinigungen).

## Revendications

1. Dans une méthode de chromatographie par écoulement en utilisant un milieu de type échangeur d'anions fort pour la séparation d'agrégats covalents et non covalents d'un matériau monomérique de type hormone de croissance qui comprend: l'équilibrage du milieu dans un tampon initial d'un pKa proche de 11,5 et d'une concentration suffisante d'un sel simple pour que le matériau monomérique de type hormone de croissance ne soit pas adsorbé sur le milieu; l'addition de la quantité appropriée de tampon et de sel simple à la solution de matériau de type hormone de croissance à purifier de manière à ce que leur concentration soit la même que dans le tampon initial; le dépôt de la solution de matériau de type hormone de croissance sur une colonne contenant le milieu et la récupération de l'effluent s'écoulant qui provient de la solution à purifier et d'une solution de déplacement par lavage utilisant le tampon initial et le lavage de la colonne avec des solutions d'une forte concentration en sel et/ou caustiques pour libérer le milieu des espèces fortement adsorbées puis la régénération du milieu en vue du cycle suivant;
l'amélioration comprenant la mise en oeuvre du procédé dans lequel le pH de la solution est supérieur à 10,5.

2. Procédé selon la revendication 1 dans lequel le pH de la solution est de 11,3 à 11,7 et le sel simple est le chlorure de sodium à une concentration 50 à 250 mM dans le tampon initial.

3. Procédé selon la revendication 3 dans lequel le tampon est la pipéridine ou un sel de phosphate.

4. Procédé selon la revendication 3 dans lequel le matériau de type hormone de croissance est la somatotropine hypophysaire bovine ou porcine.

5. Procédé selon la revendication 4 dans lequel la solution de matériau de type hormone de croissance à purifier est brute (forte teneur en impureté) ou relativement pure (faible teneur en impureté).

6. Dans une méthode chromatographique d'élution par adsorption/désorption employant un milieu de type échangeur d'anion fort pour la séparation d'agrégats covalents et non-covalents du matériau monomérique de type hormone de croissance qui comprend: l'équilibrage du milieu dans un tampon initial d'un pKa proche de 11,5 et d'une concentration appropriée d'un sel simple; l'addition de la quantité adéquate de tampon et de sel simple à la solution de matériau de type hormone de croissance à purifier de manière à ce que leur concentration soit la même que dans le tampon initial; le dépôt de la solution de matériau de type hormone de croissance sur une colonne contenant le milieu suivi d'un déplacement par lavage avec le tampon initial; l'élution du matériau monomérique de type hormone de croissance à l'aide d'une méthodologie isocratique ou utilisant un gradient; et le lavage et la régénération du milieu avec les tampons appropriés pour éliminer les espèces fortement adsorbées et le préparer en vue du cycle suivant;
l'amélioration comprenant la mise en oeuvre du procédé dans lequel le pH de la solution est supérieur à 10,5.

7. Procédé selon la revendication 6 dans lequel le pH de la solution est de 11,3 à 11,7 et le sel simple est le chlorure de sodium à une concentration 50 à 1000 mM.

8. Procédé selon la revendication 7 dans lequel le tampon est la pipéridine ou un sel de phosphate.

9. Procédé selon la revendication 8 dans lequel le matériau de type hormone de croissance est la somatotropine hypophysaire bovine ou porcine.

10. Procédé selon la revendication 9 dans lequel la solution de matériau de type hormone de croissance à purifier est brute (forte teneur en impureté) ou relativement pure (faible teneur en impureté).
